# EUROPÄISCHE PATENTSCHRIFT

(11) **EP 4 126 738 B1**
(45) Veröffentlichungstag und Bekanntmachung des Hinweises auf die Patenterteilung: **12.06.2024**
(21) Anmeldenummer: 21709446.5
(22) Anmeldetag: 09.03.2021
(51) Int. Cl.: B66B 31/02

(54) **HANDLAUFDESINFEKTIONSVORRICHTUNG FÜR PERSONENTRANSPORTANLAGEN**
HANDRAIL DISINFECTION DEVICE FOR PASSENGER CONVEYORS
DISPOSITIF DE DÉSINFECTION DE MAIN COURANTE POUR CONVOYEURS DE PASSAGERS

(30) Priorität: 25.03.2020 EP 20165562
(43) Veröffentlichungstag der Anmeldung: 08.02.2023
(73) Patentinhaber: INVENTIO AG, 6052 Hergiswil (CH)
(72) Erfinder: WAGENLEITNER, Georg, 4575 Roßleithen (AT); KLEEWEIN, Gerhard, 3021 Pressbaum (AT); BERGER, Michael, 3433 Königstetten (AT); SCHÜTZ, Richard, 1100 Wien (AT); HÄBERLE, Ulrich, 3002 Purkersdorf (AT)
(74) Vertreter: Inventio AG
(86) Internationale Anmeldenummer: PCT/EP2021/055915
(87) Internationale Veröffentlichungsnummer: WO 2021/190915

(56) Entgegenhaltungen:
- WO-A1-2019/112356
- JP-A- 2016 124 660
- KR-A- 20150 114 933
- KR-B1- 101 415 154
- US-A1- 2015 028 228

## Beschreibung

Die vorliegende Erfindung betrifft eine Handlaufdesinfektionsvorrichtung, umfassend zumindest ein Gehäuse und eine Mehrzahl von Leuchtdioden, welche keimtötende elektromagnetische Strahlung emittieren können, sowie eine Personentransportanlage mit mindestens einer Handlaufdesinfektionsvorrichtung.

Personentransportanlagen die als Fahrtreppen oder Fahrsteige ausgestalten sind, weisen in der Regel umlaufende Handläufe auf, die sich mit gleicher Geschwindigkeit bewegen wie das Transportband, auf dem die Benutzer stehen können. Dadurch können sich die Benutzer während der Fahrt an den Handläufen festhalten. Der Bereich des Handlaufs, bei dem sich die Benutzer festhalten können, wird als vorlaufendes Trum bezeichnet, während dessen rückführendes Trum vor den Benutzern der Personentransportanlage verdeckt, entgegen der Transportrichtung zurückgeführt wird. Durch die Berührung der Hände der Benutzer mit der Oberfläche der Handläufe gelangen Bakterien, Keime und Viren an den Handlauf. Die so abgelagerten Krankheitserreger können sich auf dem Handlauf vermehren und auf nachfolgende Benutzer übertragen werden. Der Handlauf einer solchen Personentransportanlage stellt somit einen unhygienischen Ort im öffentlichen Raum dar, an dem ansteckende Krankheiten übertragen werden können.

Die KR 2015 0114933 A offenbart, gemäß dem Stand der Technik, eine Sterilisationsvorrichtung für Rolltreppenhandläufe mit der bewegende Handläufe mit ultravioletten Strahlen sterilisiert werden können, die von einem Ultraviolettstrahlengenerator emittiert werden. Die Handlauf-Sterilisationsvorrichtung wird in Rolltreppen oder Fahrsteigen verwendet und am Anfang und Ende des Handlaufs oder am unteren Ende des verborgenen Bewegungspfads des Handlaufs platziert. Sie zeichnet sich dadurch aus, dass sie einen oder mehrere Generatoren für ultraviolette Strahlen umfasst, die ultraviolette Strahlen in Richtung des beweglichen Handlaufs abstrahlen. Die KR 2015 0114933 A offenbart die Präambel des Anspruchs 1.

Die WO2019/164256A1 zeigt eine Handlaufdesinfektionsvorrichtung gemäß dem Stand der Technik. Hierbei wird ein Handlauf mittels Leuchtdioden mit ultraviolettem Licht bestrahlt. Diese keimtötende elektromagnetische Strahlung zerstört die Desoxyribonukleinsäure von Viren und Bakterien, so dass auf der Oberfläche des Handlaufs ab der Austrittsseite dieser Handlaufdesinfektionsvorrichtung eine signifikante Reduktion der Mikroorganismen feststellbar ist.

Um eine noch gründlichere Desinfizierung zu ermöglichen, sieht beispielsweise die JP08188369A eine umfangreiche Vorreinigung mit rotierenden Bürsten und einer Waschanlage mit keimtötenden Fluiden vor, so dass die gereinigte Oberfläche des Handlaufs hernach durch die Strahlung der UV-Lampe desinfiziert werden kann. Allerdings ist eine solche Handlaufdesinfektionsvorrichtung sehr teuer und verursacht insbesondere wegen den erforderlichen Fluiden einen grossen Betriebsaufwand.

Aufgabe der vorliegenden Erfindung ist es daher, mittels einer kostengünstigen und einfachen Handlaufdesinfektionsvorrichtung die Krankheitserreger von den Handläufen noch sicherer unschädlich zu machen.

Diese Aufgabe wird gelöst durch eine Handlaufdesinfektionsvorrichtung, die zumindest ein Gehäuse und eine Mehrzahl von Leuchtdioden umfasst, wobei die Leuchtdioden jeweils eine Leuchtkegel-Hauptachse aufweisen, welche die Ausrichtung des Leuchtkegels der Leuchtdiode repräsentiert. Die Leuchtdioden können im Bereich ihres Leuchtkegels keimtötende elektromagnetische Strahlung emittieren. Das Gehäuse weist eine Eintrittsseite und eine der Eintrittsseite genüberliegende Austrittsseite für einen durchlaufenden Handlauf einer Personentransportanlage auf. In dem Gehäuse der Handlaufdesinfektionsvorrichtung ist zwischen der Eintrittsseite und der Austrittsseite eine mindestens in zwei Sektoren unterteilte Durchlaufstrecke für den Handlauf vorhanden. Diese Sektoren sind in der Längserstreckung der Durchlaufstrecke hintereinander angeordnet. Im ersten Sektor ist mindestens eine Leuchtdiode angeordnet, deren Leuchtkegel-Hauptachse im Wesentlichen orthogonal zu einer ersten Ebene ausgerichtet ist. Des Weiteren sind im zweiten Sektor mindestens zwei Leuchtdioden angeordnet, wobei die Leuchtkegel-Hauptachse von mindestens einer Leuchtdiode im Wesentlichen orthogonal zu einer zweiten Ebene und die Leuchtkegel-Hauptachse von mindestens einer Leuchtdiode im Wesentlichen orthogonal zu einer dritten Ebene ausgerichtet sind. Zudem sind die zweite Ebene und die dritte Ebene parallel zur Längserstreckung der Durchlaufstrecke sowie in einem Winkel von 100° bis 160° zueinander geneigt, angeordnet. Die erste Ebene ist vorzugsweise so zum Handlauf angeordnet, dass die hauptsächlich durch die Benutzer berührte Fläche durch deren Leuchtdioden möglichst direkt beschienen wird. Um eine möglichst konstante Distanz zwischen den zu desinfizierenden Oberflächen und den Leuchtdioden zu gewährleisten und damit eine konstante Desinfektionsqualität zu erreichen, weist die Handlaufdesinfektionsvorrichtung mindestens eine Führungsrolle auf, durch welche Führungsrolle der Handlauf der Personentransportanlage in der Durchlaufstrecke in einer definierten Lage durch das Gehäuse durchführbar ist.

Untersuchungen haben gezeigt, dass trotz der tödlichen elektromagnetischen Strahlung für die Mikroorganismen gewisse Überlebenschancen bestehen. Diese hängen insbesondere von der Oberflächenbeschaffenheit und der Sauberkeit des zu desinfizierenden Handlaufs ab. So können durch Risse, Spalten, Kerben, Grübchen und dergleichen mehr, sowie durch anhaftende Schmutzpartikel beschattete Zonen vorhanden sein, in denen die Mikroorganismen überleben können. Zwar können wie in der JP08188369A offenbart, durch eine mechanische Vorreinigung mit rotierenden Bürsten und eine Waschung der Handlaufoberfläche auch diese Zonen erreicht werden, jedoch hängt die Qualität der Reinigung und Desinfizierung in hohem Masse von der regelmässigen und zuverlässigen Wartung einer solchen Handlaufdesinfektionsvorrichtung ab.

Die vorliegende Erfindung geht einen anderen Weg, indem durch eine geeignete Aufteilung der Durchlaufstrecke in Sektoren sowie mit in diesen Sektoren unter verschiedenen Winkeln angeordneten, mit Leuchtdioden bestückten Ebenen, eine nahezu vollständige und in der Einwirkungsdauer ausreichende Ausleuchtung einer mit Schmutzpartikel besetzten und mit Rissen und Grübchen versehenen Handlaufoberfläche erzielt werden kann. Hierdurch kann auf eine unterhaltsintensive und teure Vorreinigung verzichtet werden. Dabei hat sich eine stumpfwinklige Anordnung von 100° bis 160° mindestens zweier Ebenen zueinander als besonders geeignet erwiesen. Als keimtötende elektromagnetische Strahlung hat sich beispielsweise ultraviolettes Licht mit einer Wellenlänge von 220nm bis 285nm als sehr effizient erwiesen. Unterstützt wird dies durch die vorteilhafte Führung des Handlaufs mittels der mindestens einer Führungsrolle, wodurch in der Durchlaufstrecke die an den Leuchtdioden vorbeiziehende Oberfläche des Handlaufs stets in gleichbleibender Lage zu diesen, geführt wird.

In einer Ausführung der Handlaufdesinfektionsvorrichtung ist an der Eintrittsseite und/ oder an der Austrittsseite mindestens eine Führungsrolle angeordnet. Die geeignete Anzahl Führungsrollen hängt im Wesentlichen von der Länge der Durchlaufstrecke und der Steifigkeit des Handlaufs ab.

In einer weiteren Ausführung der Handlaufdesinfektionsvorrichtung kann die mindestens eine Führungsrolle auch innerhalb des Gehäuses und somit in der Durchlaufstrecke angeordnet sein. Selbstverständlich sind auch Kombinationen dieser Anordnungen möglich, so dass beispielsweise jeweils eine Führungsrolle an der Eintrittsseite, an der Austrittsseite und zwischen diesen Beiden im Gehäuse eine oder mehrere Führungsrollen angeordnet sind.

Die Aufteilung in Sektoren hat noch weitere Vorteile. So erwärmt sich durch die Bestrahlung die Oberfläche des Handlaufs in Abhängigkeit von der Wellenlänge der elektromagnetischen Strahlung, dem Auftreffwinkel auf der Oberfläche und den Materialeigenschaften des Handlaufs. Eine gewisse Erwärmung ist durchaus erwünscht, da viele Viren wärmeempfindlich sind und die meisten bereits bei Temperaturen über 70° Celsius inaktiviert werden. Dank der in Sektoren aufgeteilten Durchlaufstrecke kann die Oberfläche des Handlaufs sequentiell erwärmt werden, so dass nebst der keimtötenden Strahlungswirkung eine höhere Temperatur über eine längere Zeit gehalten werden kann, ohne dass das Material des Handlaufs zu stark erwärmt wird und dadurch rascher altert. Je nach Ansteuerung der einzelnen Sektoren kann so ein gewünschtes Temperaturprofil über die ganze Durchlaufstrecke erreicht werden. Gegebenenfalls können im Material des Handlaufs, insbesondere in dessen Oberfläche, auf die elektromagnetischen Wellen abgestimmte Partikel wie beispielsweise Metallpartikel, Silberionen und dergleichen mehr eingebettet sein, die sich aufgrund der Strahlung zusätzlich erwärmen und die Wärme über einen längeren Zeitraum abgeben.

In einer weiteren Ausführung der Handlaufdesinfektionsvorrichtung ist die Neigung der zweiten Ebene zur ersten Ebene gleich gross, wie die Neigung der dritte Ebene zur ersten Ebene. Dadurch kann eine symmetrische, über die Breite des Handlaufs gleiche Ausleuchtung erzielt werden. Bei asymmetrischen Handlaufquerschnitten eignet sich gegebenenfalls auch eine asymmetrische Anordnung der zweiten und dritten Ebene zur ersten Ebene.

In einer weiteren Ausführung der Handlaufdesinfektionsvorrichtung kann in der Durchlaufstrecke ein dritter Sektor definiert sein, in dem mindestens eine Leuchtdiode angeordnet ist, deren Leuchtkegel-Hauptachse orthogonal zu einer vierten Ebene ausgerichtet ist. Der zweite Sektor ist hierbei zwischen dem ersten und dem dritten Sektor angeordnet und die vierte Ebene ist parallel zur ersten Ebene angeordnet. Dadurch kann diejenige Oberfläche, welche besonders intensiv von Benutzern berührt wird, nochmals bestrahlt werden, bevor sie die Handlaufdesinfektionsvorrichtung verlässt.

In einer weiteren Ausführung der Handlaufdesinfektionsvorrichtung können mindestens drei Leuchtdioden pro Ebene angeordnet sein, wobei pro Ebene deren Leuchtkegel-Hauptachsen im Wesentlichen parallel zueinander ausgerichtet sind. Im Wesentlichen parallel bedeutet hierbei, dass die Leuchtkegel-Hauptachsen einer Ebene räumlich um bis zu 3° von der Parallelität abweichen können. Dies kann hinsichtlich einer noch effizienteren Ausleuchtung bei sich überschneidenden Lichtkegel durchaus beabsichtigt sein. Sehr gute Ergebnisse bei geringem Energieverbrauch wurden beispielsweise mit einer Handlaufdesinfektionsvorrichtung der vorgenannten Art erzielt, die in der ersten Ebene fünf Leuchtdioden und in der zweiten und dritten Ebene jeweils drei Leuchtdioden aufweist. Durch die Anordnung einer vierten Ebene, in der vier Leuchtdioden angeordnet sind, konnte bei nur wenig erhöhtem Energieverbrauch eine hervorragende Desinfektionsleistung erzielt werden.

In einer weiteren Ausführung der Handlaufdesinfektionsvorrichtung ist das Gehäuse zweiteilig ausgeführt, wobei dieses ein U-förmiges Hauptgehäuseteil und eine, die offene Seite des Hauptgehäuseteils überspannende Abdeckung aufweist. Durch diese Aufteilung kann auf einfache Weise ein Handlauf in das Gehäuse eingelegt werden, wenn dieser aus Altersgründen ersetzt werden muss. Zudem können die Durchlaufstrecke des Gehäuses besser gereinigt und gegebenenfalls defekte Leuchtdioden einfacher ersetzt werden.

In einer weiteren Ausführung umfasst die Handlaufdesinfektionsvorrichtung einen Träger, der bezüglich der vorgesehenen Einbaulage der Handlaufdesinfektionsvorrichtung unterhalb der Durchlaufstrecke im Gehäuse angeordnet ist. An diesem Träger können alle vorhandenen Ebenen ausgebildet sein, wobei die Leuchtdioden auf diesen Ebenen befestigt sind. Hierdurch ist die Anordnung der Ebenen zueinander fest vorgegeben. Gegebenenfalls sind die Leuchtdioden auf einer oder mehreren Leiterplatten befestigt und diese auf den Ebenen angeordnet. Um die Wärme der Leuchtdioden abzuführen, können die Leiterpatte und/oder der Träger Kühlkörper aufweisen.

In einer weiteren Ausführung der Handlaufdesinfektionsvorrichtung ist mindestens ein zur Durchlaufstrecke gerichteter Reflektor zum Reflektieren der Strahlung im Gehäuse angeordnet. Hierdurch kann elektromagnetische Strahlung, die von der Handlaufoberfläche reflektiert worden ist oder am Handlauf vorbei auf die Gehäuseinnenseite trifft, auf den Handlauf gelenkt werden, um die Desinfektionsleistung nochmals zu erhöhen. Um die Reinigung des Reflektors zu vereinfachen, kann dieser vorzugsweise an der weiter oben beschriebenen Abdeckung des Gehäuses befestigt sein.

Wie bereits erwähnt, soll durch die Unterteilung in mehrere Sektoren und in diesen unterschiedlich angeordneten Ebenen eine möglichst umfassende Ausleuchtung der Oberfläche des Handlaufs erreicht werden. Dementsprechend ist es auch von Vorteil, wenn sich die Oberfläche des Handlaufs möglichst immer in gleichen Abständen zu den Ebenen durch die Durchlaufstrecke hindurchbewegt.

Handläufe für Personentransportanlagen haben üblicherweise einen C-förmigen Querschnitt, wobei der am meisten berührte und damit kontaminierteste Bereich dessen breite Rückenfläche ist. Vorzugsweise wird diese durch die Führungsrolle abgestützt und durch die auf der ersten Ebene angeordneten Leuchtdioden möglichst direkt bestrahlt, indem deren Leuchtkegel- Hauptachsen möglichst orthogonal zu diesem Bereich gerichtet sind. Um dies zu erreichen wird vorzugsweise eine Drehachse der Führungsrolle parallel zur ersten Ebene und orthogonal zur Durchlaufstrecke angeordnet.

Des Weiteren kann eine Laufflächenkontur der Führungsrolle mit einer Aussenflächenkontur des zu führenden Handlaufes korrespondieren. Hierdurch wird der Handlauf nicht nur positionspräziser durch das Gehäuse geführt. Die Führungsrolle mit einer entsprechend angepassten Kontur dient gleichzeitig als Strahlungsschutz, da durch den schmalen Spalt zwischen dem Handlauf und der Laufflächenkontur kaum noch elektromagnetische Strahlung aus dem Gehäuse austreten kann.

In einer weiteren Ausführung der Handlaufdesinfektionsvorrichtung kann der Winkel der zueinander geneigten zweiten und dritten Ebene auf die spektrale Zusammensetzung der keimtötenden elektromagnetischen Strahlung und die Materialzusammensetzung der Handlaufoberfläche abgestimmt sein. Hierbei ist insbesondere auch die Erwärmung der Oberfläche des Handlaufs durch die elektromagnetische Strahlung zu beachten, die von diesen Faktoren abhängt. Die geeigneten Winkel können unter Beachtung der gewünschten Ausleuchtung in bekannter Weise berechnet oder experimentell ermittelt werden.

Die vorangehend beschriebenen Ausführungsvarianten der Handlaufdesinfektionsvorrichtung können in Personentransportanlagen eingesetzt werden, die als Fahrtreppe oder Fahrsteig ausgebildet sind. Diese weisen mindestens eine Balustrade auf, die mindestens einen umlaufenden Handlauf umfasst. Üblicherweise weist eine Personentransportanlage der vorgenannten Art zwei Balustraden mit jeweils einem umlaufend angeordneten Handlauf auf, die beidseits eines Transportbandes der Personentransportanlage angeordnet sind. Erfindungsgemäss ist für jeden umlaufenden Handlauf einer Personentransportanlage mindestens eine Handlaufdesinfektionsvorrichtung gemäss einer der vorangehend beschriebenen Ausführungen vorgesehen.

Die Personentransportanlage kann eine Balustrade mit einem Balustradensockel umfassen, in dem ein rückführendes Trum des Handlaufs vor den Benutzern der Personentransportanlage verdeckt, geführt ist. Um die Benutzer vor den keimtötenden elektromagnetischen Strahlen bestmöglich zu schützen, kann die Handlaufdesinfektionsvorrichtung im Balustradensockel der Balustrade installiert sein. Dadurch schirmt nicht nur das Gehäuse die Umgebung vor dieser für Organismen jeglicher Art schädigender Strahlung ab, sondern als zweite Barriere auch die Verschalung des Balustradensockels.

Die Aufteilung in Sektoren bietet zudem die Möglichkeit, die Handlaufdesinfektionsvorrichtung in verschiedenen Betriebsmodi auf den Einsatzort und das Einsatzprofil der Personentransportanlage angepasst, zu betreiben. Hierfür kann ein Verfahren zum Steuern der Handlaufdesinfektionsvorrichtung in einer Steuerung der Personentransportanlage implementiert sein. Die Personentransportanlage weist einen Antriebsmotor zum Antreiben des Handlaufs und eine Antriebssteuerung zum Steuern des Antriebsmotors auf. Da die desinfizierende Wirkung der Handlaufdesinfektionsvorrichtung auch von der Durchlaufgeschwindigkeit des Handlaufs abhängt, können die Leuchtdioden der einzelnen Sektoren abhängig von einem Geschwindigkeitssignal der Antriebssteuerung an den Antriebsmotor, sektorenweise angesteuert werden.

Nachfolgend werden Ausführungsformen der Erfindung unter Bezugnahme auf die beigefügten Zeichnungen beschrieben, wobei weder die Zeichnungen noch die Beschreibung als die Erfindung einschränkend, auszulegen sind. Ferner werden für gleiche oder gleichartig wirkende Elemente dieselben Bezugszeichen verwendet. Es zeigen:
- Figur 1:: eine vereinfachte Ansicht einer als Fahrtreppe ausgestaltete Personentransportanlage mit einer Handlaufdesinfektionsvorrichtung;
- Figur 2:: ein Querschnitt entlang der Linie A-A durch die Personentransportanlage der Figur 1;
- Figur 3:: in einer Seitenansicht, die in den Figuren 1 und 2 dargestellte Handlaufdesinfektionsvorrichtung;
- Figur 4:: die Handlaufdesinfektionsvorrichtung der Figuren 1 bis 3 in einer Draufsicht;
- Figur 5:: eine dreidimensionale Ansicht der in den Figuren 1 bis 4 dargestellten Handlaufdesinfektionsvorrichtung mit einem durchgeführten Abschnitt eines Handlaufs der Personentransportanlage;
- Figur 6:: eine dreidimensionale Ansicht eines in der Handlaufdesinfektionsvorrichtung der Figuren 1 bis 5 angeordneten Trägers der mit Leuchtdioden bestückt ist;
- Figur 7:: ein Querschnitt der in den Figuren 1 bis 6 dargestellten Handlaufdesinfektionsvorrichtung entlang der in der Figur 4 eingetragenen Linie B-B;
- Figur 8:: eine mögliche Weiterbildung der der in den Figuren 1 bis 7 dargestellten Handlaufdesinfektionsvorrichtung mit Führungsrollen an deren Eintritts- und Austrittsseite.

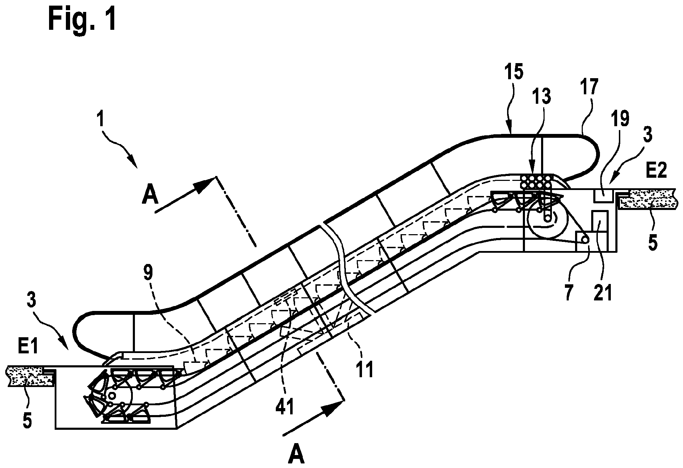

Figur 1 zeigt eine vereinfachte Ansicht einer Personentransportanlage 1 mit einem als Fachwerk ausgestalteten Tragwerk 11. Die als Fahrtreppe ausgestaltete Personentransportanlage 1 verbindet eine untere Ebene E1 mit einer oberen Ebene E2 eines Gebäudes 5. Die Personentransportanlage 1 kann über Zutrittsbereiche 3 betreten und wieder verlassen werden. Im Tragwerk 11 ist ein umlaufendes Stufenband 9 angeordnet, welches in der oberen Ebene E2 und in der unteren Ebene E1 umgelenkt wird und somit einen vorlaufenden Abschnitt und einen rücklaufenden Abschnitt aufweist. Der besseren Übersicht wegen wurde auf die detaillierte Darstellung des rücklaufenden Abschnitts verzichtet, ebenso auch auf eine detaillierte Darstellung von Spanten, Führungsschienen, und Schienenblöcken. Die Personentransportanlage 1 weist ferner zwei Balustraden 15 auf, die sich jeder Längsseite des Stufenbandes 9 entlang erstrecken, wobei in Figur 1 nur die in der Betrachtungsebene im Vordergrund angeordnete Balustrade 15 sichtbar ist. An jeder Balustrade 15 ist ein Handlauf 17 umlaufend angeordnet, wobei dessen rücklaufendes Trum in einem Balustradensockel 13 geführt ist. Dieser Balustradensockel 13 verbindet die Balustrade 15 mit dem Tagwerk 11. Mit anderen Worten wird das rückführende Trum des Handlaufs 17 vor den Benutzern der Personentransportanlage 1 verdeckt, in dem Balustradensockel 13 geführt.

Des Weiteren weist die Personentransportanlage 1 für jeden umlaufenden Handlauf 17 mindestens eine Handlaufdesinfektionsvorrichtung 41 auf. Diese ist ebenfalls im Balustradensockel 13 der Balustrade 15 installiert und damit vor den Benutzern der Personentransportanlage 1 verdeckt.

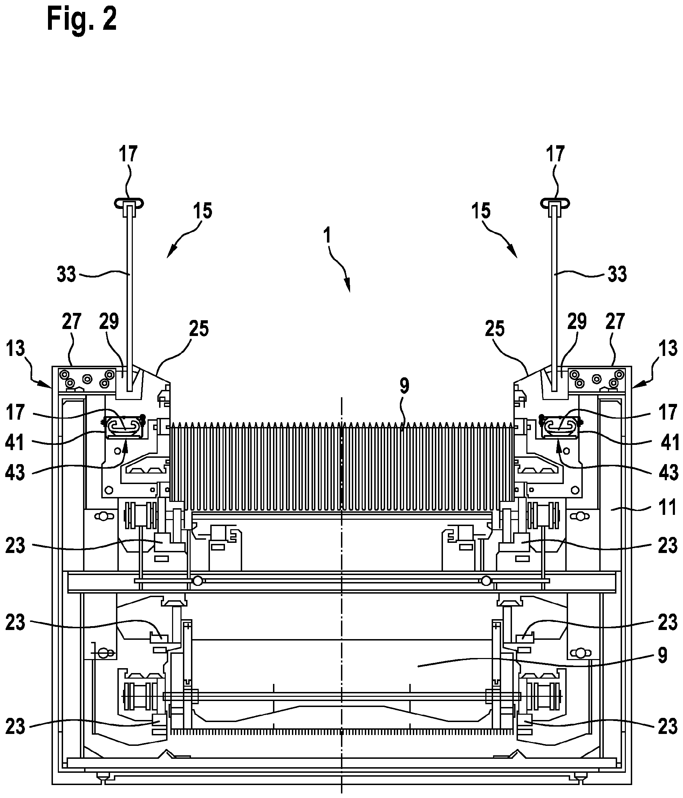

In Figur 2 ist ein Querschnitt durch die Personentransportanlage 1 gemäß Figur 1 entlang der Linie A-A dargestellt. In diesem Querschnitt ist sowohl der vorlaufende als auch der rücklaufende Abschnitt des Stufenbandes 9 sichtbar. Das Stufenband 9 wird innerhalb des Tragwerks 11 auf Führungsschienen 23 geführt. Links und rechts vom vorlaufenden, bei bestimmungsgemäßem Einbau der Personentransportanlage 1 oben angeordneten Abschnitt des Stufenbandes 9, ist je eine Balustrade 15 und ein Balustradensockel 13 angeordnet. Innerhalb des Balustradensockels 13 sind durch Verkleidungsbleche 25, 27 verdeckt, Klemmvorrichtungen 29 vorhanden, die als Klemmaufnahmen der einzelnen Balustradenpaneele 33 dienen. Hierbei ist das Balustradenpaneel 33 an seinem unteren Ende zumindest in einer der Klemmvorrichtungen 29 ortsfest eingespannt. Die Klemmvorrichtungen 29 sind entlang der Längserstreckung der Personentransportanlage 1 innerhalb des Balustradensockels 13 am Tragwerk 11 angeordnet. Unterhalb der Klemmvorrichtungen 29 ist für jeden an der Balustrade 15 umlaufend angeordnete Handlauf 17 eine Handlaufdesinfektionsvorrichtung 41 vorgesehen, die mit Leuchtdioden 43 bestückt sind, welche im Bereich ihres Leuchtkegels keimtötende elektromagnetische Strahlung emittieren können.

Da im Balustradensockel 13 auch das rücklaufende Trum des Handlaufes 17 geführt ist, ist es von Vorteil, die Handlaufdesinfektionsvorrichtung 41 im Balustradensockel 13 anzuordnen und dort das rücklaufende Trum durch diese hindurchzuführen. Dadurch kann sichergestellt werden, dass unter keinen Umständen schädigende elektromagnetische Strahlung zu den Benutzern der Personentransportanlage 1 gelangen kann.

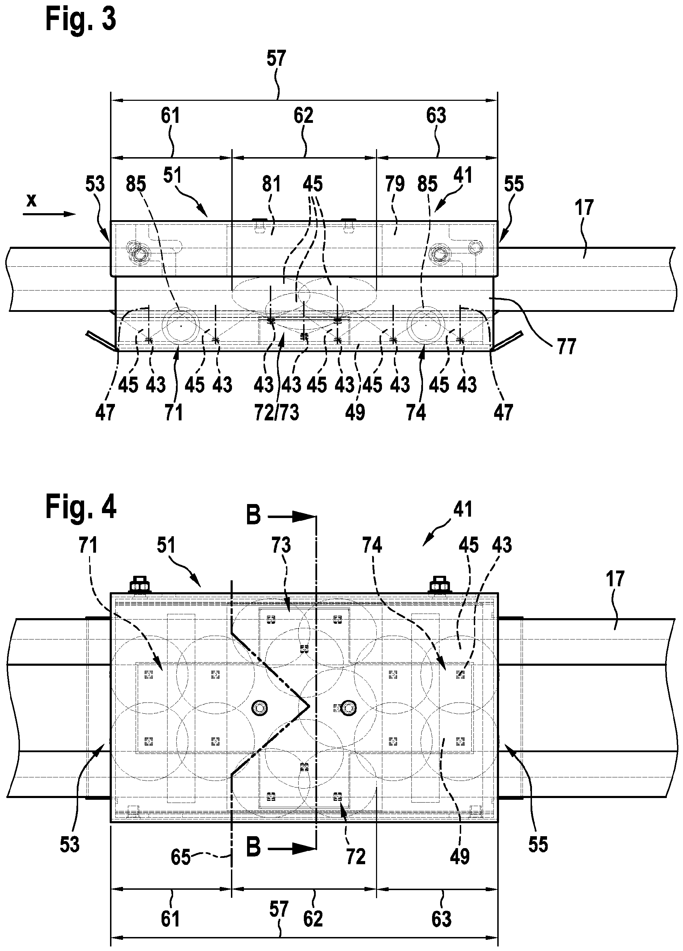

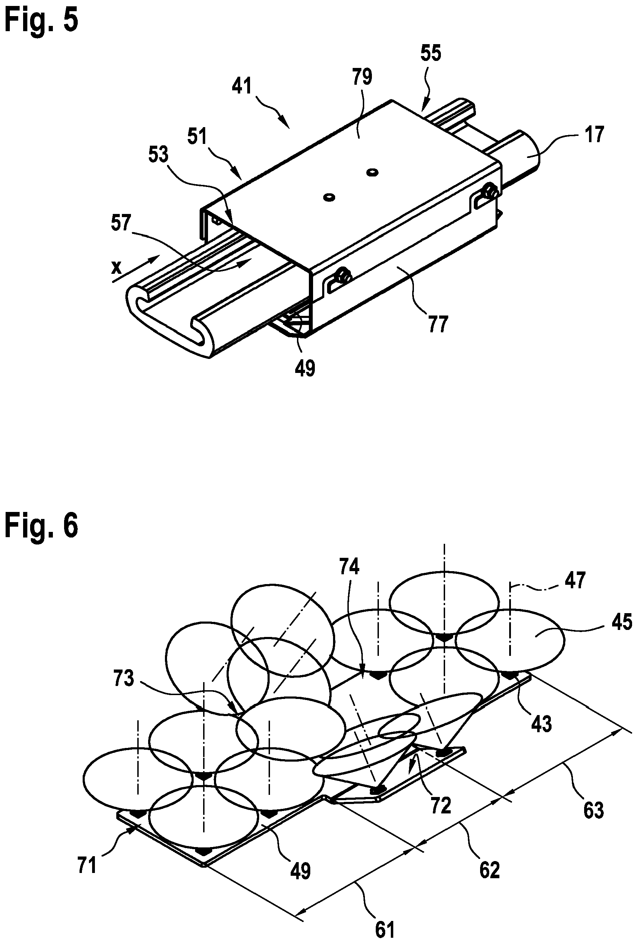

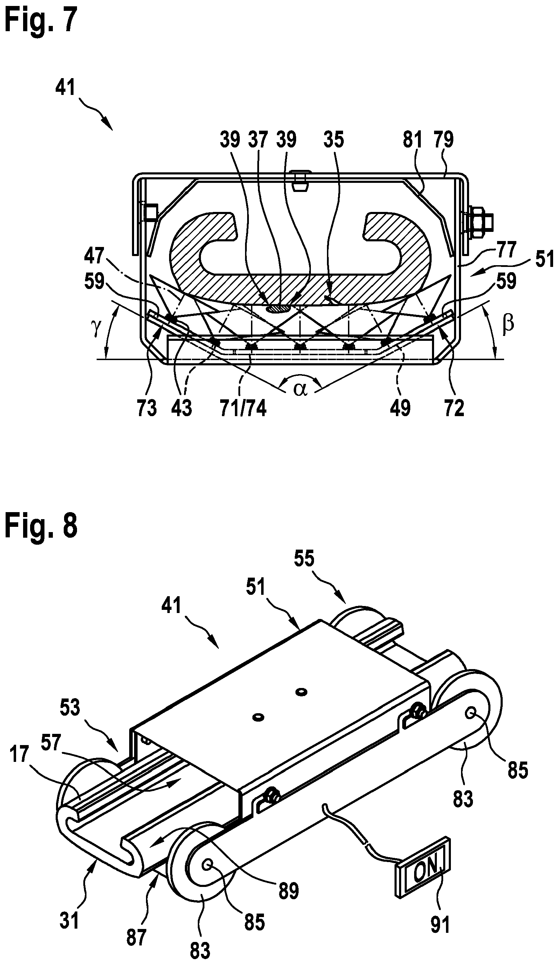

Die Figuren 3 bis 7 zeigen alle dieselbe bereits in den Figuren 1 und 2 dargestellte Handlaufdesinfektionsvorrichtung 41 oder zumindest Teile davon. Deshalb werden diese Figuren nachfolgend gemeinsam beschrieben. Genauer gesagt zeigt die Figur 3 die Handlaufdesinfektionsvorrichtung 41 in einer Seitenansicht, die Figur 4 in einer Draufsicht, die Figur 5 in einer dreidimensionalen Ansicht, die Figur 6 eine dreidimensionale Ansicht eines in der Handlaufdesinfektionsvorrichtung 41 angeordneten Trägers 49 und die Figur 7 einen Querschnitt durch die Handlaufdesinfektionsvorrichtung 41 entlang der in der Figur 4 eingetragenen Linie B-B.

Die in diesen Figuren 3 bis 7 dargestellte Handlaufdesinfektionsvorrichtung 41 umfasst ein Gehäuse 51 und eine Mehrzahl von Leuchtdioden 43. Die Leuchtdioden 43 haben konstruktionsbedingt einen Leuchtkegel 45, wobei sie im Bereich ihres Leuchtkegels 45 keimtötende elektromagnetische Strahlung emittieren können. Die räumliche Ausrichtung des Leuchtkegels 45 wird mit einer Leuchtkegel-Hauptachse 47 dargestellt, welche auch die Rotationssymmetrieachse des Leuchtkegels 45 ist. Es ist noch anzumerken, dass die eingezeichneten Leuchtkegel 45 eine kreisförmige Kontur aufweisen, die als Endfläche angesehen werden könnte. Es ist jedoch klar, dass diese Kontur keine Endfläche ist, sondern ein Leuchtkegel-Querschnitt auf beliebiger Kegelhöhe, um den Leuchtkegel besser visualisieren zu können und daher keinerlei Begrenzung der Ausbreitung elektromagnetischer Strahlung darstellen soll.

Das Gehäuse 51 weist eine Eintrittsseite 53 und eine der Eintrittsseite 53 genüberliegende Austrittsseite 55 für einen durchlaufenden Handlauf 17 einer Personentransportanlage 1 auf. Logischerweise sind die Bezeichnungen für die Eintrittsseite 53 und die Austrittsseite 55 von der Bewegungsrichtung X des Handlaufs 17 abhängig und wechseln entsprechen dieser. Im Gehäuse 51 ist zwischen der Eintrittsseite 53 und der Austrittsseite 55 eine Durchlaufstrecke 57 für den Handlauf 17 vorhanden. Diese Durchlaufstrecke 57 ist in mindestens zwei Sektoren unterteilt. Im konkreten Ausführungsbeispiel der Figuren 3 bis 7 ist die Durchlaufstrecke 57 in drei Sektoren 61, 62, 63 unterteilt. Die Sektoren 61, 62, 63 sind in der Längserstreckung der Durchlaufstrecke 57 hintereinander angeordnet und voneinander unterscheidbar ausgestaltet. Es ist noch anzumerken, dass sich die Grenzen der Sektoren 61, 62, 63 nicht zwingend nach orthogonalen Schnittebenen wie beispielsweise der in der Figur 4 eingezeichneten Schnittebene B-B richten müssen. Beispielhaft sei die strichdoppelpunktierte Sektorengrenze 65 in der Figur 4 zwischen dem ersten Sektor 61 und dem zweiten Sektor 62 erwähnt, die an die darin angeordneten Leuchtdioden 43 angepasst ist.

Im ersten Sektor 61 ist mindestens eine Leuchtdiode 43 derart angeordnet, dass deren Leuchtkegel-Hauptachse 47 im Wesentlichen orthogonal zu einer ersten Ebene 71 ausgerichtet ist. Im konkreten Ausführungsbeispiel sind insgesamt fünf Leuchtdioden im ersten Sektor 61 angeordnet, deren Leuchtkegel-Hauptachsen 47 im Wesentlichen orthogonal zur ersten Ebene71 und daher im Wesentlichen parallel zueinander angeordnet sind.

Im zweiten Sektor 62 sind mindestens zwei Leuchtdioden 43 derart angeordnet, dass die Leuchtkegel-Hauptachse 47 von mindestens einer dieser Leuchtdioden 43 im Wesentlichen orthogonal zu einer zweiten Ebene 72 und die Leuchtkegel-Hauptachse 47 von mindestens einer dieser Leuchtdioden 43 im Wesentlichen orthogonal zu einer dritten Ebene 73 ausgerichtet sind. Im konkreten Ausführungsbeispiel sind insgesamt sechs Leuchtdioden 43 im zweiten Sektor 62 angeordnet, wobei die Leuchtkegel-Hauptachsen 47 von drei dieser Leuchtdioden 43 im Wesentlichen orthogonal zur zweiten Ebene 71 beziehungsweise im Wesentlichen parallel zueinander, und die Leuchtkegel-Hauptachsen 47 der anderen drei dieser Leuchtdioden 43 im Wesentlichen orthogonal zur dritten Ebene 73 beziehungsweise im Wesentlichen parallel zueinander, angeordnet sind. Des Weiteren sind die zweite Ebene 72 und die dritte Ebene 73 parallel zur Längserstreckung der Durchlaufstrecke 57 sowie in einem Winkel α (siehe Figur 7) zueinander geneigt, angeordnet. Bei einem stumpfen Winkel α von 100° bis 160° konnten besonders gute Ergebnisse der desinfizierenden Wirkung der Handlaufdesinfektionsvorrichtung 41 erzielt werden. Insbesondere kann der Winkel α der zueinander geneigten, zweiten und dritten Ebene 72, 73 auf die spektrale Zusammensetzung der keimtötenden elektromagnetischen Strahlung und die Materialoberfläche des Handlaufes 17 abgestimmt sein.

Des Weiteren sind im Gehäuse 51 zwei Führungsrollen 85 angeordnet, so dass ein durchgeführter Handlauf 17 durch die Führungsrollen 85 abgestützt wird. Hierdurch wird die Oberfläche des Handlaufs 17 durch die auf den Ebenen 71, 72, 73 angeordneten Leuchtdioden 43 möglichst direkt bestrahlt, indem deren Leuchtkegel- Hauptachsen möglichst orthogonal zur Oberfläche des Handlaufs 17 gerichtet sind. Um dies zu erreichen wird vorzugsweise eine Drehachse der Führungsrolle 85 parallel zur ersten Ebene 71 und orthogonal zur Durchlaufstrecke 57 angeordnet.

Der Figur 7 kann auch entnommen werden, wie sich die Ausleuchtung von Problembereichen wie hinterschneidende Risse 35 und Ränder 39 von Schmutzpartikeln 37, durch die in der zweiten Ebene 72 und dritten Ebene 73 angeordneten Leuchtdioden 43 verbessern lässt.

Im dritten Sektor 63 der Durchlaufstrecke 57, ist mindestens eine Leuchtdiode 43 angeordnet, deren Leuchtkegel-Hauptachse 47 im Wesentlichen orthogonal zu einer vierten Ebene 74 ausgerichtet ist. Im konkreten Ausführungsbeispiel sind insgesamt vier Leuchtdioden 43 im dritten Sektor 63 angeordnet, deren Leuchtkegel-Hauptachsen 47 im Wesentlichen orthogonal zur vierten Ebene 74 beziehungsweise im Wesentlichen parallel zueinander angeordnet sind. Der zweite Sektor 62 ist hierbei zwischen dem ersten Sektor 61 und dem dritten Sektor 63 angeordnet und die vierte Ebene 74 ist parallel zur ersten Ebene 71 angeordnet.

Die Anordnung der Leuchtkegel-Hauptachsen 47 ist deshalb «im Wesentlichen» orthogonal beziehungsweise parallel, weil fertigungsbedingt oder gewollt, leichte Abweichungen von bis zu 3° von der Parallelität möglich sind. Wie insbesondere aus der Figur 7 ersichtlich ist, kann die Neigung β der zweiten Ebene 72 zur ersten Ebene 71 gleich gross sein, wie die Neigung γ der dritten Ebene 73 zur ersten Ebene 71.

Das Gehäuse 51 der Handlaufdesinfektionsvorrichtung 41 ist zweiteilig ausgeführt. Dieses weist ein U-förmiges Hauptgehäuseteil 77 und eine, die offene Seite des Hauptgehäuseteils 77 überspannende Abdeckung 79 auf. Hierdurch können die im Gehäuse 51 angeordneten Leuchtdioden 43 einfacher ausgetauscht, und die Durchlaufstrecke 57 besser gereinigt werden. Des Weiteren lässt sich beim Austausch eines Handlaufs 17 dieser besser aus der Handlaufdesinfektionsvorrichtung 41 entfernen beziehungsweise in deren Durchlaufstrecke 57 einfügen, ohne dass dabei eine Beschädigungsgefahr für die Leuchtdioden 43 besteht.

Wie weiter oben bereits erwähnt, kann die Handlaufdesinfektionsvorrichtung 41 einen Träger 49 umfassen (siehe insbesondere Figur 6), der bezüglich der vorgesehenen Einbaulage der Handlaufdesinfektionsvorrichtung 41 in einer Personentransportanlage 1, unterhalb der Durchlaufstrecke 57 im Gehäuse 51 angeordnet ist. Im vorliegenden Ausführungsbeispiel ist der Träger 49 im Hauptgehäuseteil 77 befestigt. An diesem Träger 49 sind vorzugsweise alle vorhandenen Ebenen 71, 72, 73, 74 ausgebildet. Die Leuchtdioden 43 sind auf diesen Ebenen befestigt. Dabei ist unerheblich, ob die Leuchtdioden 43 direkt auf dem Träger 49 befestigt sind, oder ob beispielsweise für jede Ebene 71, 72, 73, 74 eine mit den Leuchtdioden 43 bestückte Platine 59 vorhanden ist, die auf den ihnen jeweils zugeordneten Ebenen 71, 72, 73, 74 befestigt werden.

Ferner kann mindestens ein zur Durchlaufstrecke 57 gerichteter Reflektor 81 (gut ersichtlich in den Figuren 3 und 7) zum Reflektieren der von den Leuchtdioden 43 abgegebenen Strahlung im Gehäuse 51 angeordnet sein. Im vorliegenden Ausführungsbeispiel ist der Reflektor 81 in der Abdeckung 79 befestigt.

Wie bereits erwähnt, soll durch die Unterteilung in mehrere Sektoren 61, 62, 63 und durch in diesen unterschiedlich angeordneten Ebenen 71, 72, 73, 74, eine möglichst umfassende Ausleuchtung der Oberfläche des Handlaufs 17 erreicht werden. Dementsprechend ist es auch von Vorteil, wenn diese Oberfläche beziehungsweise der Handlauf 17 möglichst immer in gleichen Abständen zu den Ebenen 71, 72, 73, 74 durch die Durchlaufstrecke 57 hindurchgeführt wird.

Wie in der Figur 8 dargestellt, kann die Handlaufdesinfektionsvorrichtung 41 deshalb jeweils an der Eintrittsseite 53 und an der Austrittsseite 55 mindestens eine Führungsrolle 83 angeordnet sein. Durch diese Führungsrollen 83 lässt sich ein Handlauf 17 einer Personentransportanlage 1 in der Durchlaufstrecke 57 in einer definierten Lage durch das Gehäuse 51 durchführen.

Handläufe 17 für Personentransportanlagen 1 haben wie in der Figur 8 dargestellt, üblicherweise einen C-förmigen Querschnitt, wobei der am meisten berührte und damit kontaminierte Bereich dessen breite Rückenfläche 31 ist. Vorzugsweise wird diese durch die Führungsrolle 83 abgestützt und durch die auf der ersten Ebene 71 angeordneten Leuchtdioden 43 möglichst direkt bestrahlt, indem deren Leuchtkegel- Hauptachsen 47 möglichst orthogonal zur Rückenfläche 31 gerichtet sind. Um dies zu erreichen, ist die Drehachse 85 der Führungsrolle 83 parallel zur ersten Ebene 71 und orthogonal zur Durchlaufstrecke 57 angeordnet.

Des Weiteren kann eine Laufflächenkontur 87 der Führungsrolle 83 mit einer Aussenflächenkontur 89 des zu führenden Handlaufes 17 korrespondieren. Hierdurch wird der Handlauf 17 nicht nur positionspräziser durch das Gehäuse 51 geführt. Die Führungsrolle 83 mit einer entsprechend angepassten Laufflächenkontur 87 dient gleichzeitig als Strahlungsschutz, da durch den schmalen Spalt zwischen dem Handlauf 17 und der Laufflächenkontur 87 kaum noch elektromagnetische Strahlung aus dem Gehäuse 51 austreten kann.

Da die von den Leuchtdioden 43 emittierte keimtötende elektromagnetische Strahlung im ultravioletten Lichtbereich ist, kann ein korrektes Arbeiten der Handlaufdesinfektionsvorrichtung 41 kaum visuell erkannt werden und schon gar nicht, wenn diese im Balustradensockel 13 vor den Benutzern verborgen, installiert ist. Um den Arbeitsstatus der Handlaufdesinfektionsvorrichtung 41 anzuzeigen, kann deshalb eine geeignete Anzeigeeinrichtung 91 vorgesehen sein, die dann beispielsweise in den Zutrittsbereichen 3 (siehe Figur 1) für die Benutzer klar ersichtlich angeordnet werden kann.

Wie in der Figur 1 dargestellt ist, werden das Stufenband 9 und die Handläufe 17 durch eine Antriebseinheit 7 angetrieben. Die Antriebseinheit 7 umfasst einen Antriebsmotor 21, der durch eine Antriebssteuerung 19 der Personentransportanlage 1 angesteuert wird. Aufgrund des durch die Antriebssteuerung 19 generierten Geschwindigkeitssignals, das an einen nicht dargestellten Umrichter gesendet wird, um dementsprechend den Antriebsmotor 21 anzusteuern, ist jederzeit die aktuelle Geschwindigkeit des Handlaufs 17 in der Antriebssteuerung 19 verfügbar. Die Antriebssteuerung 19 kann nun auch dazu eingerichtet sein, die Handlaufdesinfektionsvorrichtung 41 zu betreiben. Konkret können die Leuchtdioden 43 der einzelnen Sektoren 61, 62, 63 abhängig vom Geschwindigkeitssignal der Antriebssteuerung 19 angesteuert werden, um so bei stets optimaler Desinfektionswirkung den Energieverbrauch der Leuchtdioden 43 und die schädigende Einwirkung der keimtötenden elektromagnetischen Strahlung auf das Material des Handlaufs 17 zu minimieren.

Obwohl die Figuren 1 bis 8 unterschiedliche Aspekte der vorliegenden Erfindung anhand einer als Fahrtreppe ausgestalteten Personentransportanlage 1 zeigen die vertikal voneinander beabstandete Etagen E1, E2 miteinander verbinden soll, ist es offensichtlich, dass die beschriebene Handlaufdesinfektionsvorrichtung 41 und die entsprechenden Verfahrensschritte gleichermaßen auch bei schräg angeordneten Fahrsteigen oder horizontal angeordneten Fahrsteigen verwendet werden können. Zudem können vor dem ersten Sektor 61 und/oder nach dem dritten Sektor 63 weitere Sektoren 61, 62, 63 mit mindestens einer Ebene 71, 72, 73 angeordnet sein. Selbstverständlich können pro Handlauf 17 auch mehrere Handlaufdesinfektionsvorrichtungen 41 hintereinander im Balustradensockel 13 angeordnet sein.

Abschließend ist darauf hinzuweisen, dass Begriffe wie "aufweisend", "umfassend", etc. keine anderen Elemente oder Schritte ausschließen und Begriffe wie "eine" oder "ein" keine Vielzahl ausschließen. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Die Kombinationsmöglichkeiten sind durch den nachfolgenden Anspruchssatz limitiert, welcher den Schutzumfang definiert. Bezugszeichen in den Ansprüchen sind nicht als Einschränkung anzusehen.

## Patentansprüche

1. Handlaufdesinfektionsvorrichtung (41), umfassend zumindest ein Gehäuse (51) und eine Mehrzahl von Leuchtdioden (43) mit einer Leuchtkegel-Hauptachse (47), welche Leuchtdioden (43) im Bereich ihres Leuchtkegels (45) keimtötende elektromagnetische Strahlung emittieren können, wobei das Gehäuse (51) eine Eintrittsseite (53) und eine der Eintrittsseite (53) genüberliegende Austrittsseite (55) für einen durchlaufenden Handlauf (17) einer Personentransportanlage (1) aufweist,
• wobei in dem Gehäuse (51) zwischen der Eintrittsseite (53) und der Austrittsseite (55) eine mindestens in zwei Sektoren (61, 62, 63) unterteilte Durchlaufstrecke (57) für den Handlauf (17) vorhanden ist;
• wobei die Sektoren (61, 62, 63) in der Längserstreckung der Durchlaufstrecke (57) hintereinander angeordnet sind;
• wobei im ersten Sektor (61) mindestens eine Leuchtdiode (43) angeordnet ist, deren Leuchtkegel-Hauptachse (47) im Wesentlichen orthogonal zu einer ersten Ebene (71) ausgerichtet ist; und
• wobei im zweiten Sektor (62) mindestens zwei Leuchtdioden (43) angeordnet sind, wobei die Leuchtkegel-Hauptachse (47) von mindestens einer Leuchtdiode (43) im Wesentlichen orthogonal zu einer zweiten Ebene (72) und die Leuchtkegel-Hauptachse (47) von mindestens einer Leuchtdiode (43) im Wesentlichen orthogonal zu einer dritten Ebene (73) ausgerichtet sind, und
wobei die zweite Ebene (72) und die dritte Ebene (73) parallel zur Längserstreckung der Durchlaufstrecke (57) sowie in einem Winkel (α) von 100° bis 160° zueinander geneigt, angeordnet sind, **dadurch gekennzeichnet, dass** die Handlaufdesinfektionsvorrichtung (41) mindestens eine Führungsrolle (83) aufweist, durch welche Führungsrolle (83) ein Handlauf (17) einer Personentransportanlage (1) in der Durchlaufstrecke (57) in einer zu den Leuchtdioden (43) definierten Lage durch das Gehäuse (51) durchführbar ist.

2. Handlaufdesinfektionsvorrichtung (41) nach Anspruch 1, wobei an der Eintrittsseite (53) und/oder an der Austrittsseite (55) mindestens eine Führungsrolle (83) angeordnet ist.

3. Handlaufdesinfektionsvorrichtung (41) nach Anspruch 1 oder 2, wobei mindestens eine Führungsrolle (83) innerhalb des Gehäuses (51) angeordnet ist.

4. Handlaufdesinfektionsvorrichtung (41) nach einem der Ansprüche 1 bis 3, wobei die Neigung (β) der zweiten Ebene (72) zur ersten Ebene (71) gleich gross ist, wie die Neigung (γ) der dritten Ebene (73) zur ersten Ebene (71).

5. Handlaufdesinfektionsvorrichtung (41) nach einem der Ansprüche 1 bis 4, wobei in der Durchlaufstrecke (57) ein dritter Sektor (63) definiert ist, in dem mindestens eine Leuchtdiode (43) angeordnet ist, deren Leuchtkegel-Hauptachse (47) orthogonal zu einer vierten Ebene (74) ausgerichtet ist, wobei der zweite Sektor (62) zwischen dem ersten und dem dritten Sektor (63) angeordnet ist und die vierte Ebene (74) parallel zur ersten Ebene (71) angeordnet ist.

6. Handlaufdesinfektionsvorrichtung (41) nach einem der Ansprüche 1 bis 5, wobei mindestens drei Leuchtdioden (43) pro Ebene (71, 72, 73, 74) angeordnet sind, und wobei pro Ebene (71, 72, 73, 74) deren Leuchtkegel-Hauptachsen (47) parallel zueinander ausgerichtet sind.

7. Handlaufdesinfektionsvorrichtung (41) nach Anspruch 6, wobei in der ersten Ebene (71) fünf Leuchtdioden (43), in der zweiten und dritten Ebene (72, 73) jeweils drei Leuchtdioden (43) und sofern vorhanden, in der vierten Ebene (74) vier Leuchtdioden (43) angeordnet sind.

8. Handlaufdesinfektionsvorrichtung (41) nach einem der Ansprüche 1 bis 7, wobei das Gehäuse (51) zweiteilig ausgeführt ist und ein U-förmiges Hauptgehäuseteil (77) und eine, die offene Seite des Hauptgehäuseteils (77) überspannende Abdeckung (79), aufweist.

9. Handlaufdesinfektionsvorrichtung (41) nach einem der Ansprüche 1 bis 8, wobei diese einen Träger (49) umfasst, der bezüglich der vorgesehenen Einbaulage der Handlaufdesinfektionsvorrichtung (41) unterhalb der Durchlaufstrecke (57) im Gehäuse (51) angeordnet ist, an welchem Träger (49) alle vorhandenen Ebenen (71, 72, 73, 74) ausgebildet sind und wobei die Leuchtdioden (43) auf diesen Ebenen (71, 72, 73, 74) befestigt sind.

10. Handlaufdesinfektionsvorrichtung (41) nach einem der Ansprüche 1 bis 9, wobei mindestens ein zur Durchlaufstrecke (57) gerichteter Reflektor (81) zum Reflektieren der Strahlung im Gehäuse (51) angeordnet ist.

11. Handlaufdesinfektionsvorrichtung (41) nach einem der Ansprüche 1 bis 10, wobei eine Drehachse (85) der Führungsrolle (83) parallel zur ersten Ebene (71) und orthogonal zur Durchlaufstrecke (57) angeordnet ist.

12. Handlaufdesinfektionsvorrichtung (41) nach einem der Ansprüche 1 bis 11, wobei eine Laufflächenkontur (87) der Führungsrolle (83) mit einer Aussenflächenkontur (89) des zu führenden Handlaufes (17) korrespondiert.

13. Handlaufdesinfektionsvorrichtung (41) nach einem der Ansprüche 1 bis 12, wobei der Winkel (α) der zueinander geneigten zweiten und dritten Ebene (72, 73) auf die spektrale Zusammensetzung der keimtötenden elektromagnetischen Strahlung der Leuchtdioden (43) und die Materialoberfläche des Handlaufes (17) abgestimmt ist.

14. Personentransportanlage (1) die als Fahrtreppe oder Fahrsteig ausgebildet ist, mit mindestens einer Balustrade (15), die mindestens einen umlaufenden Handlauf (17) umfasst, **dadurch gekennzeichnet, dass** die Personentransportanlage (1) für jeden umlaufenden Handlauf (17) mindestens eine Handlaufdesinfektionsvorrichtung (41) nach einem der Ansprüche 1 bis 13 aufweist.

15. Personentransportanlage (1) nach Anspruch 14, wobei die Balustrade (15) einen Balustradensockel (13) umfasst, in dem ein rückführendes Trum des Handlaufs (17) vor den Benutzern der Personentransportanlage (1) verdeckt, geführt ist, und wobei die Handlaufdesinfektionsvorrichtung (41) im Balustradensockel (13) der Balustrade (15) installiert ist.

## Claims

1. A handrail disinfecting device (41), comprising at least one housing (51) and a plurality of light-emitting diodes (43) having a main light cone axis (47), which light-emitting diodes (43) can emit germicidal electromagnetic radiation in the region of their light cone (45), wherein the housing (51) has an entry side (53) and an exit side (55) opposite the entry side (53) for a continuous handrail (17) of a passenger transport installation (1),
• wherein a pass-through segment (57) for the handrail (17), which segment is divided into at least two sectors (61, 62, 63), is present in the housing (51) between the entry side (53) and the exit side (55);
• wherein the sectors (61, 62, 63) are arranged one behind the other in the longitudinal extension of the pass-through segment (57);
• wherein at least one light-emitting diode (43) is arranged in the first sector (61), the main light cone axis (47) of which diode is oriented substantially orthogonally to a first plane (71); and
• wherein at least two light-emitting diodes (43) are arranged in the second sector (62), wherein the main light cone axis (47) of at least one light-emitting diode (43) is oriented substantially orthogonally to a second plane (72) and the main light cone axis (47) of at least one light-emitting diode (43) is oriented substantially orthogonally to a third plane (73), and
wherein the second plane (72) and the third plane (73) are arranged in parallel with the longitudinal extension of the pass-through segment (57) and so as to be mutually inclined at an angle (α) of 100° to 160°, **characterized in that** the handrail disinfecting device (41) has at least one guide roller (83), by means of which guide roller (83) a handrail (17) of a passenger transport installation (1) can be guided through the housing (51) in the pass-through segment (57) in a defined position in relation to the light-emitting diodes (43).

2. The handrail disinfecting device (41) according to claim 1, wherein at least one guide roller (83) is arranged on the entry side (53) and/or on the exit side (55).

3. The handrail disinfecting device (41) according to claim 1 or 2, wherein at least one guide roller (83) is arranged inside the housing (51).

4. The handrail disinfecting device (41) according to any of claims 1 to 3, wherein the inclination (β) of the second plane (72) relative to the first plane (71) is the same as the inclination (γ) of the third plane (73) relative to the first plane (71).

5. The handrail disinfecting device (41) according to any of claims 1 to 4, wherein a third sector (63) is defined in the pass-through segment (57), in which sector at least one light-emitting diode (43) is arranged, the main light cone axis (47) of which is oriented orthogonally to a fourth plane (74), wherein the second sector (62) is arranged between the first and the third sector (63), and the fourth plane (74) is arranged in parallel with the first plane (71).

6. The handrail disinfecting device (41) according to any of claims 1 to 5, wherein at least three light-emitting diodes (43) are arranged in each plane (71, 72, 73, 74), and wherein their main light cone axes (47) are oriented in parallel with one another in each plane (71, 72, 73, 74).

7. The handrail disinfecting device (41) according to claim 6, wherein five light-emitting diodes (43) are arranged in the first plane (71), three light-emitting diodes (43) each are arranged in the second and the third plane (72, 73) and four light-emitting diodes (43) are arranged in the fourth plane (74), if present.

8. The handrail disinfecting device (41) according to any of claims 1 to 7, wherein the housing (51) is designed in two parts and has a U-shaped main housing part (77) and a cover (79) spanning the open side of the main housing part (77).

9. The handrail disinfecting device (41) according to any of claims 1 to 8, wherein said handrail disinfecting device comprises a carrier (49), which is arranged in the housing (51) below the pass-through segment (57) with respect to the intended installation position of the handrail disinfecting device (41), on which carrier (49) all planes (71, 72, 73, 74) present are formed, and wherein the light-emitting diodes (43) are fastened to said planes (71, 72, 73, 74).

10. The handrail disinfecting device (41) according to any of claims 1 to 9, wherein at least one reflector (81) which is directed toward the pass-through segment (57) and is intended for reflecting the radiation is arranged in the housing (51).

11. The handrail disinfecting device (41) according to any of claims 1 to 10, wherein an axis of rotation (85) of the guide roller (83) is arranged in parallel with the first plane (71) and orthogonally to the pass-through segment (57).

12. The handrail disinfecting device (41) according to any of claims 1 to 11, wherein a tread contour (87) of the guide roller (83) corresponds to an outer surface contour (89) of the handrail (17) to be guided.

13. The handrail disinfecting device (41) according to any of claims 1 to 12, wherein the angle (α) of the mutually inclined second and third plane (72, 73) is matched to the spectral composition of the germicidal electromagnetic radiation of the light-emitting diodes (43) and the material surface of the handrail (17).

14. A passenger transport installation (1) that is designed as an escalator or a moving walkway, comprising at least one balustrade (15) that comprises at least one circulating handrail (17), **characterized in that** the passenger transport installation (1) has at least one handrail disinfecting device (41) according to any of claims 1 to 13 for each circulating handrail (17).

15. The passenger transport installation (1) according to claim 14, wherein the balustrade (15) comprises a balustrade base (13) in which a returning run of the handrail (17) is guided so as to be concealed from the users of the passenger transport installation (1), and wherein the handrail disinfecting device (41) is installed in the balustrade base (13) of the balustrade (15).

## Revendications

1. Dispositif de désinfection de main courante (41), comprenant au moins un boîtier (51) et une pluralité de diodes électroluminescentes (43) comportant un axe principal de cône de lumière (47), lesquelles diodes électroluminescentes (43) peuvent émettre un rayonnement électromagnétique germicide dans la zone de leur cône de lumière (45), dans lequel le boîtier (51) présente un côté d'entrée (53) et un côté de sortie (55) opposé au côté d'entrée (53) pour une main courante (17) continue d'une installation de transport de personnes (1),
• dans lequel une voie de passage (57) pour la main courante (17), laquelle voie de passage est divisée en au moins deux secteurs (61, 62, 63), est présente dans le boîtier (51) entre le côté d'entrée (53) et le côté de sortie (55) ;
• dans lequel les secteurs (61, 62, 63) sont disposés l'un derrière l'autre dans l'extension longitudinale de la voie de passage (57) ;
• dans lequel au moins une diode électroluminescente (43) est disposée dans le premier secteur (61), l'axe principal de cône de lumière (47) de ladite diode étant orienté sensiblement orthogonalement à un premier plan (71) ; et
• dans lequel au moins deux diodes électroluminescentes (43) sont disposées dans le deuxième secteur (62), dans lequel l'axe principal de cône de lumière (47) d'au moins une diode électroluminescente (43) est orienté sensiblement orthogonalement à un deuxième plan (72) et l'axe principal de cône de lumière (47) d'au moins une diode électroluminescente (43) est orienté sensiblement orthogonalement à un troisième plan (73), et
dans lequel le deuxième plan (72) et le troisième plan (73) sont disposés parallèlement à l'extension longitudinale de la voie de passage (57) et de manière inclinée l'un par rapport à l'autre selon un angle (α) allant de 100° à 160°, **caractérisé en ce que** le dispositif de désinfection de main courante (41) présente au moins un rouleau de guidage (83), au moyen duquel rouleau de guidage (83) une main courante (17) d'une installation de transport de personnes (1) peut être passée à travers le boîtier (51) dans la voie de passage (57) dans une position définie par rapport aux diodes électroluminescentes (43).

2. Dispositif de désinfection de main courante (41) selon la revendication 1, dans lequel au moins un galet de guidage (83) est disposé sur le côté d'entrée (53) et/ou sur le côté de sortie (55).

3. Dispositif de désinfection de main courante (41) selon la revendication 1 ou 2, dans lequel au moins un galet de guidage (83) est disposé à l'intérieur du boîtier (51).

4. Dispositif de désinfection de main courante (41) selon l'une des revendications 1 à 3, dans lequel l'inclinaison (β) du deuxième plan (72) par rapport au premier plan (71) est égale à l'inclinaison (γ) du troisième plan (73) par rapport au premier plan (71).

5. Dispositif de désinfection de main courante (41) selon l'une des revendications 1 à 4, dans lequel un troisième secteur (63) est défini dans la voie de passage (57), dans lequel troisième secteur est disposée au moins une diode électroluminescente (43) dont l'axe principal de cône de lumière (47) est orienté orthogonalement à un quatrième plan (74), dans lequel le deuxième secteur (62) est disposé entre le premier et le troisième secteur (63) et le quatrième plan (74) est disposé parallèlement au premier plan (71).

6. Dispositif de désinfection de main courante (41) selon l'une des revendications 1 à 5, dans lequel au moins trois diodes électroluminescentes (43) sont disposées par plan (71, 72, 73, 74), et dans lequel, par plan (71, 72, 73, 74), les axes principaux de cônes de lumière (47) desdites diodes sont orientés parallèlement les uns aux autres.

7. Dispositif de désinfection de main courante (41) selon la revendication 6, dans lequel cinq diodes électroluminescentes (43) sont disposées dans le premier plan (71), respectivement trois diodes électroluminescentes (43) sont disposées dans les deuxième et troisième plans (72, 73) et, si elles sont présentes, quatre diodes électroluminescentes (43) sont disposées dans le quatrième plan (74).

8. Dispositif de désinfection de main courante (41) selon l'une des revendications 1 à 7, dans lequel le boîtier (51) est conçu en deux parties et présente une partie de boîtier principale (77) en forme de U et un élément de recouvrement (79) couvrant le côté ouvert de la partie de boîtier principale (77).

9. Dispositif de désinfection de main courante (41) selon l'une des revendications 1 à 8, dans lequel ledit dispositif comprend un support (49) qui, par rapport à la position de montage prévue du dispositif de désinfection de main courante (41), est disposé en dessous de la voie de passage (57) dans le boîtier (51), sur lequel support (49) sont réalisés tous les plans (71, 72, 73, 74) présents, et dans lequel les diodes électroluminescentes (43) sont fixées sur lesdits plans (71, 72, 73, 74).

10. Dispositif de désinfection de main courante (41) selon l'une des revendications 1 à 9, dans lequel au moins un réflecteur (81) orienté vers la voie de passage (57) est disposé dans le boîtier (51) pour réfléchir le rayonnement.

11. Dispositif de désinfection de main courante (41) selon l'une des revendications 1 à 10, dans lequel un axe de rotation (85) du galet de guidage (83) est disposé parallèlement au premier plan (71) et orthogonalement à la voie de passage (57).

12. Dispositif de désinfection de main courante (41) selon l'une des revendications 1 à 11, dans lequel un contour de surface de roulement (87) du galet de guidage (83) correspond à un contour de surface extérieure (89) de la main courante (17) à guider.

13. Dispositif de désinfection de main courante (41) selon l'une des revendications 1 à 12, dans lequel l'angle (α) des deuxième et troisième plans (72, 73) inclinés l'un par rapport à l'autre est adapté à la composition spectrale du rayonnement électromagnétique germicide des diodes électroluminescentes (43) et à la surface du matériau de la main courante (17).

14. Installation de transport de personnes (1) réalisée sous forme d'escalier roulant ou de trottoir roulant, comportant au moins une balustrade (15) qui comprend au moins une main courante (17) périphérique, **caractérisée en ce que** l'installation de transport de personnes (1) présente, pour chaque main courante (17) périphérique, au moins un dispositif de désinfection de main courante (41) selon l'une des revendications 1 à 13.

15. Installation de transport de personnes (1) selon la revendication 14, dans laquelle la balustrade (15) comprend un socle de balustrade (13) dans lequel est guidé, de manière à être caché des utilisateurs de l'installation de transport de personnes (1), un brin de retour de la main courante (17), et dans lequel le dispositif de désinfection de main courante (41) est installé dans le socle de balustrade (13) de la balustrade (15).
